(19) Europäisches Patentamt
European Patent Office
Office européen des brevets

(11) EP 2 207 821 B1

(12) EUROPEAN PATENT SPECIFICATION

(45) Date of publication and mention
of the grant of the patent:
**25.05.2011 Bulletin 2011/21**

(21) Application number: **08846255.1**

(22) Date of filing: **06.11.2008**

(51) Int Cl.:
***C08F 210/02*** (2006.01)

(86) International application number:
**PCT/EP2008/065090**

(87) International publication number:
**WO 2009/060044 (14.05.2009 Gazette 2009/20)**

(54) **PROCESS FOR PRODUCING (ULTRA) HIGH MOLECULAR WEIGHT POLYETHYLENE**

VERFAHREN ZUR HERSTELLUNG VON POLYETHYLEN MIT (ULTRA)HOHEM MOLEKULARGEWICHT

PROCÉDÉ DE PRODUCTION DE POLYÉTHYLÈNE DE MASSE MOLÉCULAIRE (ULTRA) HAUTE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **06.11.2007 EP 07120053**

(43) Date of publication of application:
**21.07.2010 Bulletin 2010/29**

(73) Proprietor: **DSM IP Assets B.V.**
**6411 TE Heerlen (NL)**

(72) Inventors:
• **KIDD, Timothy James**
**NL-6224 LH Maastricht (NL)**
• **JANSSEN, Robert Hendrik Catharina**
**NL-6191PC Beek (NL)**
• **FREDERIX, Filip**
**Heverlee 3001 (NL)**
• **STOLK, Jan**
**NL-6135 HJ Sittard (NL)**
• **SMELT, Harold Jan**
**NL-6132 CX Sittard (NL)**

(74) Representative: **Scheltus, Irma**
**DSM Intellectual Property**
**P.O. Box 4**
**6100 AA Echt (NL)**

(56) References cited:
**EP-A- 0 273 654          WO-A-2007/065152**
**US-A1- 2005 146 070**

**Description**

The present invention relates to a process for producing an (ultra) high molecular weight polyethylene ((U) HMWPE) article. The present invention further relates to an article obtainable by said process, and to the use of said (U)HMWPE in a medical application.

Excellent properties in terms of wear, fatigue and fracture resistance have made (U)HMWPE the material of choice in orthopedics, especially the fabrication of articular components for arthroplasty, for which a high wear resistance is required. The acetabular cup or liner in a total hip joint replacement and the tibial insert in a total knee joint replacement are important applications of (U)HMWPE.

HMWPE is herein defined as a substantially linear ethylene homopolymer or copolymer with a weight average molecular weight (Mw) of $3.10^5$ g/mol or more, a molecular weight distribution ($M_w/M_n$) of between 2 and 18 and an intrinsic viscosity (IV) of 1,5-8 dl/g. Preferably, the IV of HMWPE is 3-8 dl/g and more preferably, 5-8 dl/g. The IV is defined according to ISO 1628-3. UHMWPE is herein defined as a substantially linear ethylene homopolymer or copolymer with a weight average molecular weight (Mw) of $10^6$ g/mol or more, a molecular weight distribution ($M_w/M_n$) of between 2 and 18 and an IV of 8 dl/g or more. Preferably, the IV of UHMWPE is between 8 and 60 dl/g.

(U)HMWPE can be obtained by any known process for the production of (U)HMWPE, as described by for example Steven M. Kurtz in "The UHMWPE Handbook", Elsevier Academic Press, 2004, p. 14-22. (U)HMWPE is generally obtained as a powder which can further be processed by molding and machining as described below.

Studies have shown that cross-linking of (U)HMWPE with gamma or electron beam rays is highly effective against wear, which was most clearly demonstrated for smooth counterfaces, such as those generally involved in a prosthetic coupling.

However, despite the outstanding success of the use of (cross-linked) (U)HMWPE in total joint replacement surgery, failures arising from, for example, aseptic loosening or mechanical failure of the component after few years of implantation are still quite frequent, as shown in J.H. Dumbleton et al., J. Arthroplasty 2002, 17(5): 649-661 and T.W. Bauer et al., Skeletal Radiol 1999, 28(9): 483-497. It has been demonstrated that most of the failures were to be ascribed to the wear of the (U)HMWPE component. Wear is a major concern, since wear leads to the formation of debris, which in turn induces an inflammatory response, causing loosening of the implant.

It has been found that the failure of the components due to the oxidative stability of (U)HMWPE is at least partly due to side-effects of irradiation during cross-linking and/or sterilization. Irradiation was found to induce, in addition to cross-linking which has a positive effect on the wear properties, oxidative degradation of polyethylene. Oxidative degradation has a negative effect on the mechanical properties of (U)HMWPE.

The degree of said oxidative degradation strongly depends on the irradiation dosage, which should therefore be kept as low as possible. This will result in lower amounts of radicals formed and thus reduces the need for annealing and/or stabilizers, which is the focus of the current device manufacturers. On the other hand, the dosage needs to be high enough to allow a sufficient cross-link density.

In Bracco et al., Polymer 46 (2005) 10648-10657 ("Bracco") additives were added to (U)HMWPE to enhance the sensitivity of (U)HMWPE for cross-linking, with the aim to achieve sufficient cross-link density using a lower irradiation dose. (U)HMWPE specimens soaked in 1,7-octadiene, methyl acetylene and ethylene, respectively, were irradiated with electron beam radiation to different doses in single or multiple passages and studied using Fourier Transform Infrared (FTIR) spectroscopy. Tensile tests were performed with all samples in order to monitor changes in the mechanical properties. Gel fraction measurements proved that cross-linking took place in all the irradiated samples, but 1,7-octadiene turned out to be the most effective additive for the present purpose, exhibiting a good efficiency in enhancing cross-linking.

A disadvantage of the above procedure is that the additives need to be incorporated in the (U)HMWPE in a separate step by means of diffusion. The diffusion process is very slow and even when using thin films of (U)HMWPE the process takes 7 days, as shown in 2.3 of the experimental section in Bracco. It is clear that the process would take even longer when artificial joints are used.

The procedure disclosed in U.S. Patent No. 5,594,041, also involving diffusion of an additive into a polymer, for example (U)HMWPE, and subsequent cross-linking, suffers from the same disadvantage. In fact, because of the slow diffusion rate, the additive only diffuses to a limited depth, as stated in column 3, I. 49-67. Another problem of the methods used in Bracco and in U.S. Patent No. 5,594,041 is that any non-converted polyene will remain in the final article and may cause regulatory issues depending on the use.

A primary object of the invention is therefore to at least provide an alternative process for the production of cross-linked (U)HMWPE. In particular it is an object of the invention to provide a process for the production of cross-linked (U)HMWPE which is more efficient than the processes disclosed in the prior art.

Surprisingly it was found that the object of the invention can be reached by a process for the production of an (ultra) high molecular weight polyethylene ((U)HMWPE) article comprising:

-   copolymerizing ethylene with a linear, branched or cyclic polyene having 3 to 100 carbon atoms, resulting in a copolymer of ethylene and polyene ((U)HMWPE-P), using such a content of polyene that the number of polyene branches in (U)HMWPE-P is 0.01 to 15 on the average per 1000 carbon atoms;

- cross-link the (U)HMWPE-P during or after molding the (U)HMWPE-P.

In a first embodiment of the invention the process for the production of an (ultra) high molecular weight polyethylene ((U)HMWPE) article can comprise the following steps:

a) copolymerizing ethylene with a linear, branched or cyclic polyene having 3 to 100 carbon atoms, resulting in a copolymer of ethylene and polyene (hereinafter referred to as (U)HMWPE-P), using such a content of polyene that the number of polyene branches in (U)HMWPE-P is 0.01 to 15 on the average per 1000 carbon atoms;
b) molding the (U)HMWPE-P into a stock shape or an article comprising (U)HMWPE-P;
c) cross-linking the stock shape or the article via gamma radiation or electron beam radiation, resulting in a stock shape or an article comprising cross-linked (U)HMWPE-P ((U)HMWPE-P-X);
d) optionally, machining the stock shape into an article;

in which step c) and step d) can be performed in either order.

In a second embodiment of the invention the process for the production of an (ultra) high molecular weight polyethylene ((U)HMWPE) article can comprise the following steps:

a) copolymerizing ethylene with a linear, branched or cyclic polyene having 3 to 100 carbon atoms, resulting in a copolymer of ethylene and polyene (hereinafter referred to as (U)HMWPE-P), using such a content of polyene that the number of polyene branches in (U)HMWPE-P is 0.01 to 15 on the average per 1000 carbon atoms;
b) adding an initiator, preferably a peroxide, and optionally a coagent;
c) molding the (U)HMWPE-P into a stock shape or an article comprising cross-linked (U)HMWPE-P ((U)HMWPE-P-X);
d) optionally, when further cross-linking via gamma radiation or electron beam radiation is applied, cross-linking the stock shape or the article comprising (U)HMWPE-P-X using gamma radiation or electron beam radiation, resulting in a stock shape or an article comprising further cross-linked (U)HMWPE-P-X;
e) optionally, if step c or d) results in a stock shape, machining the stock shape into an article;

in which step d) and step e) can be performed in either order.

The above process is more efficient than the process described in the prior art as the additives used according to the prior art are now incorporated as a comonomer during the polymerization process such that no extra diffusion step involving an additive is necessary. The fact that polyene can be copolymerized with ethylene is even more surprising in view of the comment in Bracco on page 10650, left column: "the amount of pendent double bonds cannot be increased during (U)HMWPE synthesis". Moreover, in the process according to the invention, a substantial amount of any non-converted polyene is likely to be removed in step a), e.g when removing the dispersant in a slurry process as described below. A lower amount of non-converted polyene in the article is an advantage in view of regulatory issues. The oxidative stability of (U)HMWPE-P-X is substantially higher than that of cross-linked (U)HMWPE homopolymer. Moreover (U)HMWPE-P-X possesses improved mechanical properties compared to cross-linked (U)HMWPE homopolymer.

In the description (U)HMWPE-P is defined as a copolymer of ethylene with a linear, branched or cyclic polyene having 3-100 carbon atoms and (U)HMWPE-P-X is defined as a crosslinked copolymer of ethylene with a linear, branched or cyclic polyene having 3-100 carbon atoms.

The (U)HMWPE-P can be obtained by any known process for the production of (U)HMWPE (step a), for example, by slurry-copolymerizing ethylene and the linear branched or cyclic polyene (hereinafter referred to as polyene) as the comonomer in an organic suspension agent in the presence of a catalyst comprising a transition metal of the group IVb, Vb, VIb or VIII of the periodic table, a halide of a metal of the group I, II or III of the periodic table or an organic metal compound, using such a content of polyene that the number of polyene branches is 0.01 to 15 on the average per 1000 carbon atoms. In particular a Ziegler-Natta catalyst made of titanium chloride, optionally on a support, for example $MgCl_2$ or $SiO_2/MgCl_2$, and organo-aluminum compounds, e.g. triethyl-aluminum or diethyl aluminum chloride, is used. Alternatively a metallocene catalyst may be used, as described in for example EP-1605000A1. Because these chemicals are very sensitive to air and moisture, all the steps in the synthesis are carried out in an inert gas environment. Polymerization generally takes place at a temperature of between 55 and 90˚C under a pressure of between 1 and 40 bar. The molecular weight of the (U)HMWPE-P can be controlled by adding very small amounts of hydrogen to the ethylene gas or by changing the polymerization temperature, the pressure or the amount of organo-aluminum compound. After the polymerization, the polymer particles are separated from the suspension agent and dried. In particular, most of the suspension agent is separated via centrifugation, and the remaining suspension agent is removed by drying, for example in a stirred bed dryer or a fluid bed dryer. As a suspension agent medium boiling aliphatic solvents can be used, for example hexane or heptane. The boiling point of said suspension agent is preferably higher than the reaction temperature but not too high to avoid problems when removing the suspension agent.

The polyene used in the present invention is a linear, branched or cyclic polyene with 3-100 carbon atoms, preferably with 3-50 carbon atoms and more preferably with 3-20 carbon atoms. The polyene used in the present invention is a hydrocarbon compound having in the molecule at least two unsaturated bonds, preferably double bonds. For example, there can be mentioned non-conjugated diene type hydrocarbon compounds such as 1,4-pentadiene, 1,4-hexadiene, 2,4-hexadiene, 1,5-hexadiene, 1,6-heptadiene, 1,7-octadiene, 1,2-propadiene, 2,5-dimethyl-1,5-hexadiene, 4-methyl-1,4-hexadiene, 5-methyl-1,4-hexadiene, 4-ethyl-1,4-hexadiene, 4,5-dimethyl-1,4-hexadiene, cyclohexadiene, 4-methyl-1,4-heptadiene, 4-ethyl-1,4-heptadiene, 5-methyl-1,4-heptadiene, 4-ethyl-1,4-octadiene, 5-methyl-1,4-octadiene and 4-n-propyl-1,4-decadiene, conjugated polyolefin type hydrocarbon compounds such as 1,3,5-hexatriene, 1,3,5,7-octatetraene and 2-vinyl-1,3-butadiene, and non-conjugated polyolefin type hydrocarbon compounds such as squalene, divinylbenzene, vinylnorbornene, ethylene norbornene and dicyclopentadiene. Among them, non-conjugated diene type hydrocarbon compounds having an especially excellent copolymerizability with ethylene are preferred.

The determination of the polyene content can be done by using an infrared spectrophotometer. The absorbances at 880, 910 and 965 $cm^{-1}$, which indicated the double bonds in the polyene structure included in the ethylene chain, are measured, and the measured values are converted to the number of the unsaturations per 1000 carbon atoms by using a calibration curve prepared in advance by using a model compound in $^{13}C$ nuclear magnetic resonance spectroscopy. The sum of the converted values of the peaks, which differ according to the structure of the introduced polyene, indicates the total polyene content. Alternatively, $^{1}H$ and/or $^{13}C$-NMR can be applied to determine the content of unsaturations.

The polyene comonomer should be used in such an amount that the polyene content per 1000 carbon atoms in the polymer chain is within the above-mentioned range. If the polyene content is smaller than 0.01 polyene branches per 1000 carbon atoms, a structure effective for improving the wear resistance cannot be formed. On the contrary, if the polyene content exceeds 15 polyene branches per 1000 carbon atoms, the crystallinity is drastically reduced and a high elastic modulus cannot be obtained. Preferably the polyene comonomer should be used in such an amount that the polyene content per 1000 carbon atoms in the polymer chain is between 0.01 and 10, more preferably between 0.01 and 5, in particular between 0.01 and 3 polyene branches per 1000 carbon atoms.

Several processing methods can be used for molding and, if necessary, machining of (U)HMWPE-P into bulk products, as described in for example Steven M. Kurtz in "The UHMWPE Handbook", Elsevier Academic Press, 2004, p. 22-31 (hereinafter "The UHMWPE Handbook").

The first method is compression molding, wherein a mold filled with (U)HMWPE-P is subjected to a combination of high temperature and high pressure for a certain amount of time. Subsequently the system is cooled at a slow and uniform rate in order to minimize shrinkage and deformation, and optimize the crystallinity and the mechanical properties of the product. The product is than machined into smaller blocks or cylindrical bars from which the final components, for example articular components, can be machined.

The second method, ram extrusion, produces cylindrical bar stocks ranging in diameter from 25 mm to 150 mm. In this process, the (U)HMWPE-P charge is fed into a channel and then heat is applied. A ram then compresses and extrudes the plasticized charge into the heated cylindrical barrel, where it is consolidated into a cylindrical bar stock. As the ram moves back and forward, the stock in the chamber is refilled. The final components, for example articular components, can be machined from the bar stock.

In the third method, direct compression molding, the (U)HMWPE-P charge is consolidated into a final or semifinal bulk product using a pre-shaped mold. Machining is not always necessary when this method is applied. Although this process is slow and costly, orthopedic articular components made using this method have very smooth surface finishes and excellent dimensional consistency.

A HMWPE powder can also be melt processed by injection molding or extrusion into a sheet or a bar. In this way the end product can be directly obtained, but machining the product to obtain the end product is also possible.

In addition to the above methods Hot Isostatic Pressing (HIPing) can be applied, as described in The UHMWPE Handbook on p. 27.

Machining of (U)HMWPE-P consists of milling and turning operations for both first rough and finishing steps. More details about machining are provided in The UHMWPE Handbook on p. 31-32.

Three methods that are used for cross-linking (U)HMWPE articles, and which can also be applied to the article comprising (U)HMWPE-P, are gamma irradiation, electron beam irradiation and chemical-induced cross-linking, as described by G. Lewis in Biomaterials 2001, 22: 371-401.

With gamma irradiation or electron beam irradiation the irradiation dose used to obtain a highly cross-linked (U)HMWPE article is chosen between 30 and 250 kGray (kGy), preferably between 30 and 170 kGy and more preferably between 40 and 130 kGy. To obtain a lower cross-linked (U)HMWPE article or when irradiation is used in combination with chemical cross-linking by the use of an initiator, a lower irradiation dose can be used, from for instance 25 to 50 kGy.

For sterilization of the (U)HMWPE article according to the invention an irradiation dose between 10 and 40 kGy, preferably between 20 and 35 kGy can be used. Either the stock shape or the article or both, can be subjected to irradiation cross-linking by applying electron beam or gamma radiation. According to a second embodiment of the invention the (U)HMWPE is cross-linked by adding an initiator, for example a peroxide, and optionally a coagent to the (U)HMWPE. Optionally a coagent, a compound with 2 or more unsaturations, is used to enhance the peroxide cross-linking efficiency. Examples of suitable peroxides include tert-butyl cumyl peroxide, tert-butyl peroxybenzoate, di-tert-butyl peroxide, 3,3,5,7,7-pentamethyl-1,2,4-trioxepane,1,1-di(tert-butylperoxy)-3,3,5-trimethylcyclohexane, butyl 4,4-di (tert-butylperoxy)valerate, 2,5-dimethyl-2,5-di(tert-butylperoxy)hexyne-3, 2,5-dimethyl-2,5-di(tert-butylperoxy)hexane, di(4-methylbenzoyl) peroxide, dibenzoyl peroxide, di(2,4-dichlorobenzoyl) peroxide, dicumyl peroxide, 3,3,5,7,7-pentamethyl-1,2,4-trioxepane, 1-(2-tert.-butylperoxyisopropyl)-3-isopropenyl benzene, 2,4-diallyloxy-6-tert-butylperoxy-1,3,5-triazine, di(tert-butylperoxyisopropyl)benzene, diisopropylbenzene monohydroperoxide, cumyl hydroperoxide, and tert-butyl hydroperoxide. Examples of suitable coagents include divinylbenzene, diallylphthalate, triallylcyanurate, triallylisocyanuarate, triallyltrimellitate, meta-phenylene bismaelimide, ethyleneglycol dimethacrylate, ethyleneglycol diacrylate, trimethylopropane timethacrylate, trimethylopropane, timethacrylate, pentaerytritol tetramethacrylate, zinc diacrylate, zinc dimethacrylate, and polybutadiene. These peroxides and coagents, either as liquid or powder, can be mixed with the (U)HMWPE-P powder, and then consolidated by molding. Whereas when irradiation is used cross-linking occurs in the solid state of (U)HMWPE-P, peroxide cross-linking occurs in the melt.

In addition to ethylene and, for (U)HMWPE-P and (U)HMWPE-P-X, polyene, (U)HMWPE, (U)HMWPE-P and (U)HMWPE-P-X may comprise one or more alpha-olefin comonomers, for example propylene, butene, pentene, hexene, 4-methylpentene or octene, to achieve improved processing characteristics or alter the physical and mechanical properties of the polymer. Furthermore the polymer can contain non-polymer materials such as additives, solvents and fillers. In particular, anti-oxidants, such as vitamine E and Hindered Amine Light Stabilizers (HALS) may be added to avoid excessive oxidation during cross-linking, sterilization or use.

A much lower amount of the HALS stabilizer can be used compared with the amount of vitamin E. The HALS stabilizer is preferably used in an amount of between 0.001 and 5 % by weight, more preferably between 0.01 and 2 % by weight, most preferably between 0.02 and 1 % by weight, based on the total weight of the (U)HMWPE. Preferably, the HALS stabilizer chosen is a compound derived from a substituted piperidine compound, in particular any compound which is derived from an alkyl-substituted piperidyl, piperidinyl or piperazinone compound or a substituted alkoxypiperidinyl compound.

Also, an inorganic stearate such as calcium or zinc stearate may be added to the (U)HMWPE-P as a flow agent or to minimize the effect of any catalyst residues, which have a potential for corroding the conversion machines. Moreover, calcium stearate may act as a lubricant when a part is to be fabricated using ram-extrusion of the polymer and may help the product to maintain its white color.

The article comprising (U)HMWPE-P-X according to the invention may be sterilized applying for example gamma sterilization in air or, preferably, in an inert atmosphere, using a radiation dose of between 10 and 40 kGy, gas plasma sterilization or ethylene oxide gas sterilization, as described in the above-referenced "The UHMWPE Handbook" on p. 37-47.

The article comprising (U)HMWPE-P-X according to the invention can be applied in a wide variety of applications, for example in medical applications, such as in orthopedics as bearing material in artificial joints. (U)HMWPE-P-X can be used in for example hip arthroplasty, knee replacements, shoulder replacements and spinal applications such as total disc replacement. These applications are described in detail in the above-referenced "The UHMWPE Handbook" in Chapters 4-6 (hip), 7-8 (knee), 9 (shoulder) and 10 (spinal applications).

The present invention will now be described in detail with reference to the following examples that by no means limit the scope of the invention.

EXAMPLES

Materials:

Catalyst;
Ti-containing Ziegler-Natta catalysts A and B
Triethylalumina (TEA); Chemtura

Dienes:
1,7-Octadiene; Aldrich 99%
Vinylnorbornene (VNB); INEOS > 99%

Examples 1 -8 and Comparative Experiments A and B

Preparation of an ethylene- 1, 7-octadiene and ethylene-vinylnorbornene copolymers and the characterization thereof.

Polymerization conditions:

    Polymerizations were performed in a 2L SS autoclave.
    Polymerization temperature: 60˚C, Monomer pressure: 0.5 MPa
    Co-monomer amount - 10 ml and 40 ml
    Scavenger: TEA 1.3 mmol

Polymerization procedure

The batch polymerization was performed in a 2.0 L batch autoclave equipped with an mechanical stirrer. The reaction temperature was set to 60˚C and controlled by a thermostat. The feed streams (solvent and ethylene) were purified by various absorption media to remove catalyst killing impurities such as water, oxygen and polar compounds as is known to someone skilled in the art. During polymerization ethylene was continuously fed to the gas cap of the reactor. The pressure of the reactor was kept constant at 0.5 MPa by a back-pressure valve.
In an inert atmosphere the previously dried reactor was filled with 800 mL heptane. After the solvent had reached the desired temperature, the second monomer (diene) and the catalyst and TEA were added. Ethylene was added at a flow of 96 nL/h to a maximum pressure of 0.5 MPa. After the desired polymerization time, the contents of the reactor were collected, filtered and washed with 3 L heptane to remove the un-reacted diene. After drying under vacuum at 50˚C overnight, the polymer was weighed and samples were prepared for analysis.
Polymer powder was compression molded into test samples according to ISO-11542. Irradiation of the test samples was performed by gamma irradiation (dose 25 kGy/hour) on samples that were vacuum sealed into paper bags with an aluminum coating on the inside. To prepare the test samples for the swell test and the wear test a stock sample was prepared that was irradiated and was later machined into the small test samples needed for those tests.

Swell test

The swell ratio tests were performed according to the ASTM F2214-02 standard. Blocks of the 5mm*5mm*5mm dimensions were machined and subjected to swelling in o-xylene.

Wear test

Pins with a diameter of 9 mm and a length of 12 mm were machined from the irradiated stock sample.
The wear test was performed in a Weartester SuperCTPOD TE87. In the weartester 100 pins could be evaluated at once. The pins were moved in the tester with a circular motion.
The pins were tested for a total distance of 18 km at 1 Hz cycle frequency in HyClone Alpha Calf serum (from Thermofischer Scientific) that was diluted with ultra-pure water (1:1). The serum was used for lubrication and the pins were moved in the weartester against polished CoCr disks using 1.1 MPa nominal contact pressure and 31.4 mm/s sliding speed. To retard the harmful degradation of the lubricant, its temperature was kept at $20 \pm 0.5$ ˚C. At intervals of 6 km, the test was stopped, and the specimens and their holders were dismantled and cleaned. The pins were vacuum desiccated, allowed to stabilize in room atmosphere for at least 2 hours and then weighed, after which the system was reassembled, and the test was continued with fresh lubricant. For all samples, a soak control pin was used to see if the materials showed considerable weight gain due to fluid absorption.
The wear factor was determined using the formulae below:

$$Wear_y = \left\{ \left( m_{pin_{run(x)}} - m_{pin_{run(0)}} \right) + \left( m_{pin_{soak(x)}} - m_{pin_{soak(0)}} \right) \right\}_{pin_y} \rightarrow [mg]$$

$x = [1,2,3] \rightarrow$ *number · of · run*
$y = [1,2,3,4,5] \rightarrow$ *number · pin · per · material*

$$Wearfactor_y = 1000 \cdot \left\{ \frac{\left( \frac{wear_y}{\rho} \right)}{(F \cdot s_{r,m,})} \right\} \rightarrow \left[ 10^{-6}\, mm^3 / Nm \right]$$

$x = [1,2,3] \rightarrow number \cdot of \cdot run$

$y = [1,2,3,4,5] \rightarrow number \cdot pin \cdot per \cdot material$

$F = Load \cdot on \cdot pins \rightarrow [N]$

$s = Distance \cdot per \cdot run \rightarrow [m]$   $\rho = Density \cdot of \cdot material \rightarrow \left[ \frac{mg}{mm^3} \right]$

The value given in Table 1 is the average value of 5 samples.

Amount of double bonds

The amount of double bonds per 1000 carbon atoms was determined by using an infrared spectrophotometer. The absorbances at 880, 910 and 965 cm$^{-1}$, which indicate the double bonds in the polyene structure included in the ethylene chain, were measured, and the measured values were converted to the number of the unsaturations per 1000 carbon atoms by using a calibration curve prepared in advance by using a model compound in $^{13}$C nuclear magnetic resonance spectroscopy. The sum of the converted values of the peaks, which differed according to the structure of the introduced polyene, indicated the total polyene content.

Table 1. Examples 1-8 and Comparative Experiments A and B

| Example | Diene Type | Amount (ml) | Catalyst | Catalyst Activity gpol/ gcat*hr*bar | C=C chain end per 1000 C | wear factor (10$^6$ mm$^3$/Nm | swell ratio |
|---|---|---|---|---|---|---|---|
| A | -- | 0 | A | 3972 | -- | 3.35 | 3.9 |
| 1 | VNB | 10 | A | 1394 | 0.07 | 1.43 | 3.2 |
| 2 | VNB | 20 | A | 1621 | 0.11 | 1.27 | 3.2 |
| 3 | Octadiene | 10 | A | 1013 | 0.06 | 1.60 | 3.5 |
| 4 | Octadiene | 20 | A | 1540 | 0.09 | 1.09 | 3.1 |
| B | -- | 0 | B | 522 | -- | 2.34 | 3.5 |
| 5 | VNB | 10 | B | 369 | 0.12 | 1.21 | 3.2 |
| 6 | VNB | 20 | B | 152 | 0.14 | 0.95 | 3.1 |
| 7 | Octadiene | 10 | B | 532 | 0.08 | 1.05 | 3.0 |
| 8 | Octadiene | 20 | B | 532 | 0.12 | 0.63 | 2.8 |

From the results for examples 1-8 it became clear that:

- a higher amount of diene is incorporated in the copolymer when the amount of diene added during polymerization is higher,
- the crosslink density (calculated from the swell ratio) increases when a higher amount of diene is incorporated in the copolymer,
- the wear factor decreases (lower wear) significantly with an increasing amount of diene, incorporated in the copolymer.

**Claims**

1.  Process for the production of an (ultra) high molecular weight polyethylene ((U)HMWPE) article comprising:

       - copolymerizing ethylene with a linear, branched or cyclic polyene having 3 to 100 carbon atoms, resulting in a copolymer of ethylene and polyene ((U)HMWPE-P), using such a content of polyene that the number of polyene branches in (U)HMWPE-P is 0.01 to 15 on the average per 1000 carbon atoms;
- cross-link the (U)HMWPE-P during or after molding the (U)HMWPE-P.

2. Process according to claim 1 comprising the following steps:

a) copolymerizing ethylene with a linear, branched or cyclic polyene having 3 to 100 carbon atoms, resulting in a copolymer of ethylene and polyene (hereinafter referred to as (U)HMWPE-P), using such a content of polyene that the number of polyene branches in (U)HMWPE-P is 0.01 to 15 on the average per 1000 carbon atoms;
b) molding the (U)HMWPE-P into a stock shape or an article comprising (U)HMWPE-P;
c) cross-linking the stock shape or the article via gamma radiation or electron beam radiation, resulting in a stock shape or an article comprising cross-linked (U)HMWPE-P ((U)HMWPE-P-X);
d) optionally, machining the stock shape into an article; in which step c) and step d) can be performed in either order.

3. Process according to claim 1 comprising the following steps:

a) copolymerizing ethylene with a linear, branched or cyclic polyene having 3 to 100 carbon atoms, resulting in a copolymer of ethylene and polyene (hereinafter referred to as (U)HMWPE-P), using such a content of polyene that the number of polyene branches in (U)HMWPE-P is 0.01 to 15 on the average per 1000 carbon atoms;
b) adding an initiator, preferably a peroxide, and optionally a coagent;
c) molding the (U)HMWPE-P into a stock shape or an article comprising cross-linked (U)HMWPE-P ((U)HMWPE-P-X);
d) optionally, when further cross-linking via gamma radiation or electron beam radiation is applied, cross-linking the stock shape or the article comprising (U)HMWPE-P-X using gamma radiation or electron beam radiation, resulting in a stock shape or an article comprising further cross-linked (U)HMWPE-P-X;
e) optionally, if step c or d) results in a stock shape, machining the stock shape into an article;

in which step d) and step e) can be performed in either order.

4. Process according to anyone of claims 1-3, wherein the polyene is a non-conjugated diene type hydrocarbon compound.

5. Process according to claim 4, wherein the non-conjugated diene type hydrocarbon compound is chosen from the group consisting of 1,4-pentadiene,1,5-hexadiene, 1,6-heptadiene, 1,7-octadiene, 2,5-dimethyl-1,5-hexadiene, 4-methyl-1,4-hexadiene, 5-methyl-1,4-hexadiene, 4-ethyl-1,4-hexadiene, 4,5-dimethyl-1,4-hexadiene, 4-methyl-1,4-heptadiene, 4-ethyl-1,4-heptadiene, 5-methyl-1,4-heptadiene, 4-ethyl-1,4-octadiene, 5-methyl-1,4-octadiene, 4-n-propyl-1,4-decadiene, squalene, and divinylbenzene, vinylnorbornene, ethylene norbornene and dicyclopentadiene.

6. Process according to any one of claims 1-5, wherein the number of polyene branches in (U)HMWPE-P is 0.01 to 5 on the average per 1000 carbon atoms.

7. Process according to any one of claims 1-6, wherein the (U)HMWPE is UHMWPE with an intrinsic viscosity of 8 dl/g or more.

8. Process according to any one of claims 1-7, wherein (U)HMWPE-P is molded by injection molding, extrusion, compression molding, ram-extrusion, direct compression molding or hot isostatic pressing.

9. Process according to any one of claims 1-8, wherein the article comprising (U)HMWPE-P is cross-linked using gamma radiation.

10. Article comprising (U)HMWPE-P-X obtainable by the process according to any one of claims 1-9.

11. Article according to claim 10, further comprising a hindered amine light stabilizer (HALS).

12. Article according to claim 10 or 11, wherein the article is an artificial medical implant.

13. Article according to claim 12, wherein the artificial medical implant is used for hip arthroplasty, for example as acetubular cup or liner in a total hip joint replacement, knee replacement, for example as the tibial insert in a total knee joint replacement, shoulder replacement or spinal applications such as total disc replacement.

14. Article according to claim 12, wherein the artificial medical implant is a total joint replacement.

15. Use of the article according to any one of claims 10-14 in artificial medical implant, more in particular in total joint replacement

**Patentansprüche**

1. Verfahren zur Herstellung eines Gegenstands aus Polyethylen mit (ultra)hohem Molekulargewicht ((U)HMWPE), umfassend:

   - Copolymerisieren von Ethylen mit einem linearen, verzweigten oder cyclischen Polyen mit 3 bis 100 Kohlenstoffatomen, um ein Copolymer von Ethylen und Polyen ((U)HMWPE-P) zu ergeben, unter Verwendung eines derartigen Gehalts an Polyen, dass die Anzahl an Polyenzweigen in dem (U)HMWPE-P im Mittel 0,01 bis 15 pro 1000 Kohlenstoffatomen beträgt;
   - Vernetzen des (U)HMWPE-P während oder nach Formen des (U)HMWPE-P.

2. Verfahren gemäß Anspruch 1, umfassend folgende Schritte:

   a) Copolymerisieren von Ethylen mit einem linearen, verzweigten oder cyclischen Polyen mit 3 bis 100 Kohlenstoffatomen, um ein Copolymer von Ethylen und Polyen (nachstehend (U)HMWPE-P genannt) zu ergeben, unter Verwendung eines derartigen Gehalts an Polyen, dass die Anzahl an Polyenzweigen in dem (U)HMWPE-P im Mittel 0,01 bis 15 pro 1000 Kohlenstoffatomen beträgt;
   b) Formen des (U)HMWPE-P zu einer Stangenform oder einem Gegenstand, umfassend (U)HMWPE-P;
   c) Vernetzen der Stangenform oder des Gegenstands durch Gammastrahlung oder Elektronenstrahlung, um eine Stangenform oder einen Gegenstand zu ergeben, umfassend vernetztes (U)HMWPE-P ((U)HMWPE-P-X);
   d) gegebenenfalls Verarbeiten der Stangenform zu einem Gegenstand;

   wobei Schritt c) und Schritt d) in beliebiger Reihenfolge durchgeführt werden können.

3. Verfahren gemäß Anspruch 1, umfassend folgende Schritte:

   a) Copolymerisieren von Ethylen mit einem linearen, verzweigten oder cyclischen Polyen mit 3 bis 100 Kohlenstoffatomen, um ein Copolymer von Ethylen und Polyen (nachstehend (U)HMWPE-P genannt) zu ergeben, unter Verwendung eines derartigen Gehalts an Polyen, dass die Anzahl an Polyenzweigen in dem (U)HMWPE-P im Mittel 0,01 bis 15 pro 1000 Kohlenstoffatomen beträgt;
   b) Zugeben eines Initiators, vorzugsweise eines Peroxids, und gegebenenfalls eines Koagens;
   c) Formen des (U)HMWPE-P zu einer Stangenform oder einem Gegenstand, umfassend vernetztes (U)HMWPE-P ((U)HMWPE-P-X);
   d) gegebenenfalls, wenn weiteres Vernetzen durch Gammastrahlung oder Elektronenstrahlung angewendet wird, Vernetzen der Stangenform oder des Gegenstands, umfassend (U)HMWPE-P-X, unter Verwendung von Gammastrahlung oder Elektronenstrahlung, um eine Stangenform oder einen Gegenstand zu ergeben, umfassend weiter vernetztes (U)HMWPE-P-X;
   e) gegebenenfalls, falls Schritt c) oder d) eine Stangenform ergibt, Verarbeiten der Stangenform zu einem Gegenstand;

   wobei Schritt d) und Schritt e) in beliebiger Reihenfolge durchgeführt werden können.

4. Verfahren gemäß einem der Ansprüche 1-3, wobei das Polyen eine nichtkonjugierte Kohlenwasserstoffverbindung vom Dientyp ist.

5. Verfahren gemäß Anspruch 4, wobei die nichtkonjugierte Kohlenwasserstoffverbindung vom Dientyp ausgewählt ist aus der Gruppe, bestehend aus 1,4-Pentadien, 1,5-Hexadien, 1,6-Heptadien, 1,7-Octadien, 2,5-Dimethyl-1,5-hexadien, 4-Methyl-1,4-hexadien, 5-Methyl-1,4-hexadien, 4-Ethyl-1,4-hexadien, 4,5-Dimethyl-1,4-hexadien, 4-Methyl-1,4-heptadien, 4-Ethyl-1,4-heptadien, 5-Methyl-1,4-heptadien, 4-Ethyl-1,4-octadien, 5-Methyl-1,4-octadien, 4-n-Propyl-1,4-decadien, Squalen und Divinylbenzol, Vinylnorbornen, Ethylennorbornen und Dicyclopentadien.

6. Verfahren gemäß einem der Ansprüche 1-5, wobei die Anzahl der Polyenzweige in dem (U)HMWPE-P im Mittel 0,01 bis 5 pro 1000 Kohlenstoffatome beträgt.

7. Verfahren gemäß einem der Ansprüche 1-6, wobei das (U)HMWPE UHMWPE mit einer inhärenten Viskosität von 8 dl/g oder mehr ist.

8. Verfahren gemäß einem der Ansprüche 1-7, wobei (U)HMWPE-P durch Spritzgießen, Extrusion, Formpressen, Sinterextrusion, direktes Formpressen oder isostatisches Warmpressen geformt wird.

9. Verfahren gemäß einem der Ansprüche 1-8, wobei der Gegenstand, der (U)HMWPE-P umfasst, unter Verwendung von Gammastrahlung vernetzt wird.

10. Gegenstand, umfassend (U)HMWPE-P-X, erhältlich durch das Verfahren gemäß einem der Ansprüche 1-9.

11. Gegenstand gemäß Anspruch 10, ferner umfassend einen gehindertes-Amin-Lichtstabilisator (HALS).

12. Gegenstand gemäß Anspruch 10 oder 11, wobei der Gegenstand ein künstliches medizinisches Implantat ist.

13. Gegenstand gemäß Anspruch 12, wobei das künstliche medizinische Implantat für die Hüftarthroplastik, beispielsweise als Hüftgelenkpfanne oder Liner in einem vollständigen Hüftgelenkersatz, Knieersatz, beispielsweise als der Tibiaeinsatz bei einem vollständigen Kniegelenkersatz, Schulterersatz oder für Spinalanwendungen, wie z. B. vollständigen Bandscheibenersatz, verwendet wird.

14. Gegenstand gemäß Anspruch 12, wobei das künstliche medizinische Implantat ein vollständiger Gelenkersatz ist.

15. Verwendung des Gegenstands gemäß einem der Ansprüche 10-14 als künstliches medizinisches Implantat, insbesondere bei vollständigem Gelenkersatz.

**Revendications**

1. Procédé pour la production d'un article en polyéthylène à (ultra-)haut poids moléculaire ((U)HMWPE) comprenant :

   - la copolymérisation d'éthylène avec un polyène linéaire, ramifié ou cyclique portant 3 à 100 atomes de carbone, pour obtenir un copolymère d'éthylène et de polyène ((U)HMWPE-P), en utilisant une teneur en polyène telle que le nombre de ramifications polyène dans le (U)HMWPE-P est de 0,01 à 15 en moyenne pour 1000 atomes de carbone ;
   - la réticulation du (U)HMWPE-P pendant ou après le moulage du (U)HMWPE-P.

2. Procédé selon la revendication 1 comprenant les étapes suivantes :

   a) copolymérisation d'éthylène avec un polyène linéaire, ramifié ou cyclique portant 3 à 100 atomes de carbone, pour obtenir un copolymère d'éthylène et de polyène (désigné ci-après (U)HMWPE-P), en utilisant une teneur en polyène telle que le nombre de ramifications polyène dans le (U)HMWPE-P est de 0,01 à 15 en moyenne pour 1000 atomes de carbone ;
   b) moulage du (U)HMWPE-P en un produit semi-fini ou un article comprenant du (U)HMWPE-P ;
   c) réticulation du produit semi-fini ou de l'article par rayons gamma ou faisceau d'électrons, pour obtenir un produit semi-fini ou un article comprenant du (U)HMWPE-P réticulé ((U)HMWPE-P-X) ;
   d) éventuellement, usinage du produit semi-fini en un article ;

   dans lequel l'étape c) et l'étape d) peuvent être exécutées dans n'importe quel ordre.

3. Procédé selon la revendication 1 comprenant les étapes suivantes :

   a) copolymérisation d'éthylène avec un polyène linéaire, ramifié ou cyclique portant 3 à 100 atomes de carbone, pour obtenir un copolymère d'éthylène et de polyène (désigné ci-après (U)HMWPE-P), en utilisant une teneur en polyène telle que le nombre de ramifications polyène dans le (U)HMWPE-P est de 0,01 à 15 en moyenne pour 1000 atomes de carbone ;

b) addition d'un initiateur, de préférence un peroxyde, et éventuellement d'un co-agent ;

c) moulage du (U)HMWPE-P en un produit semi-fini ou un article comprenant du (U)HMWPE-P réticulé ((U) HMWPE-P-X) ;

d) éventuellement, lorsqu'une réticulation supplémentaire par rayons gamma ou faisceau d'électrons est appliquée, réticulation du produit semi-fini ou de l'article comprenant du (U)HMWPE-P-X par rayons gamma ou faisceau d'électrons, pour obtenir un produit semi-fini ou un article comprenant du (U)HMWPE-P-X davantage réticulé ;

e) éventuellement, si l'étape c) ou d) aboutit à un produit semi-fini, usinage du produit semi-fini en un article ;

dans lequel l'étape d) et l'étape e) peuvent être exécutées dans n'importe quel ordre.

4. Procédé selon l'une quelconque des revendications 1 à 3, dans lequel le polyène est un composé hydrocarboné de type diène non conjugué.

5. Procédé selon la revendication 4, dans lequel le composé hydrocarboné de type diène non conjugué est choisi dans le groupe constitué par le 1,4-pentadiène, le 1,5-hexadiène, le 1,6-heptadiène, le 1,7-octadiène, le 2,5-diméthyl-1,5-hexadiène, le 4-méthyl-1,4-hexadiène, le 5-méthyl-1,4-hexadiène, le 4-éthyl-1,4-hexadiène, le 4,5-diméthyl-1,4-hexadiène, le 4-méthyl-1,4-heptadiène, le 4-éthyl-1,4-heptadiène, le 5-méthyl-1,4-heptadiène, le 4-éthyl-1,4-octadiène, le 5-méthyl-1,4-octadiène, le 4-n-propyl-1,4-décadiène, le squalène, le divinylbenzène, le vinylnorbornène, l'éthylène-norbornène et le dicyclopentadiène.

6. Procédé selon l'une quelconque des revendications 1 à 5, dans lequel le nombre de ramifications polyène dans le (U)HMWPE-P est de 0,01 à 5 en moyenne pour 1000 atomes de carbone.

7. Procédé selon l'une quelconque des revendications 1 à 6, dans lequel le (U)HMWPE est un UHMWPE avec une viscosité intrinsèque de 8 dl/g ou plus.

8. Procédé selon l'une quelconque des revendications 1 à 7, dans lequel le (U)HMWPE-P est moulé par moulage par injection, extrusion, moulage par compression, extrudo-sintérisation, moulage par compression directe, ou compression isostatique à chaud.

9. Procédé selon l'une quelconque des revendications 1 à 8, dans lequel l'article comprenant du (U)HMWPE-P est réticulé en utilisant des rayons gamma.

10. Article comprenant du (U)HMWPE-P-X pouvant être obtenu par le procédé selon l'une quelconque des revendications 1 à 9.

11. Article selon la revendication 10, comprenant en outre un photostabilisant à base d'amine stériquement encombrée (HALS).

12. Article selon la revendication 10 ou 11, l'article étant un implant médical artificiel.

13. Article selon la revendication 12, l'implant médical artificiel étant utilisé pour l'arthroplastie de la hanche, par exemple comme tasse acétabulaire ou doublure dans un remplacement total de l'articulation de la hanche, le remplacement du genou, par exemple comme insert tibial dans un remplacement total de l'articulation du genou, le remplacement de l'épaule, ou des applications spinales telles qu'un remplacement total de disque.

14. Article selon la revendication 12, l'implant médical artificiel étant un remplacement total d'articulation.

15. Utilisation de l'article selon l'une quelconque des revendications 10 à 14 dans un implant médical artificiel, plus particulièrement dans un remplacement total d'articulation.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5594041 A

- EP 1605000 A1

### Non-patent literature cited in the description

- **Steven M. Kurtz.** The UHMWPE Handbook. Elsevier Academic Press, 2004, 14-22
- **J.H. Dumbleton et al.** *J. Arthroplasty,* 2002, vol. 17 (5), 649-661
- **T.W. Bauer et al.** *Skeletal Radiol,* 1999, vol. 28 (9), 483-497

- **Bracco et al.** *Polymer,* 2005, vol. 46, 10648-10657
- **Steven M. Kurtz.** The UHMWPE Handbook. Elsevier Academic Press, 2004, 22-31
- **G. Lewis.** *Biomaterials,* 2001, vol. 22, 371-401
- The UHMWPE Handbook. knee, 7-8